# EUROPÄISCHE PATENTSCHRIFT

(11) **EP 1 872 809 B1**
(45) Veröffentlichungstag und Bekanntmachung des Hinweises auf die Patenterteilung: **26.10.2011**
(21) Anmeldenummer: 07010844.4
(22) Anmeldetag: 01.06.2007
(51) Int. Cl.: A61L 31/08, A61L 31/16, A61L 27/54, A61L 27/28

(54) **Implantat mit einer Cholesterol- oder Cholesterolester-haltigen Beschichtung**
Implant with a coating containing cholesterol or cholesterol ester
Implant doté d'un revêtement contenant du cholestérol ou des esters de cholestérol

(30) Priorität: 26.06.2006 DE 102006029247
(43) Veröffentlichungstag der Anmeldung: 02.01.2008
(73) Patentinhaber: Biotronik VI Patent AG, 6341 Baar (CH)
(72) Erfinder: Korzuschnik, Ellen, 91077 Hetzles (DE); Borck, Alexander, Dr., 91086 Aurachtal (DE)
(74) Vertreter: Lindner-Vogt, Karin L.

(56) Entgegenhaltungen:
- WO-A-01/15752
- US-A1- 2004 224 003
- US-A1- 2006 030 936

## Beschreibung

### Technisches Gebiet

Die Erfindung betrifft ein beschichtetes Implantat sowie eine neue Verwendung von Cholesterol oder eines Cholesterolesters.

### Hintergrund der Erfindung und Stand der Technik

Implantate unterschiedlichster Ausprägung sind seit vielen Jahrzehnten fester Bestandteil der Medizintechnik.

Zum Beispiel hat sich die Implantation von Stents als eine der wirkungsvollsten therapeutischen Maßnahmen bei der Behandlung von Gefäßerkrankungen etabliert. Stents haben den Zweck, im Inneren des Körpers eines Patienten eine Stützfunktion zu übernehmen. Dementsprechend sind Stents implantierbar ausgeführt und besitzen eine Tragstruktur, die die Stützfunktion gewährleistet. Bekannt sind Implantate aus metallischen Werkstoffen. Die Wahl von Metallen als Werkstoff für die Tragstruktur eines derartigen Implantats beruht vor allem auf den mechanischen Eigenschaften von Metallen.

Metallische Stents sind in großer Zahl bekannt. Einer der Hauptanwendungsbereiche solcher Stents ist das dauerhafte Weiten und Offenhalten von Gefäßverengungen, insbesondere von Verengungen (Stenosen) der Herzkranzgefäße. Daneben sind auch Aneurysmenstents bekannt, die eine Stützfunktion für eine beschädigte Gefäßwand bieten. Derartige Stents besitzen in der Regel eine Umfangswandung von ausreichender Tragkraft, um das verengte Gefäß im gewünschten Maße offen zu halten. Um einen ungehinderten Blutfluss durch den Stent zu ermöglichen, ist dieser an beiden Stirnseiten offen. Kompliziertere Ausführungen ermöglichen auch einen ungehinderten Blutfluss in Nebengefäße. Die tragende Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. An den Stent wird daher grundsätzlich die Anforderung gestellt, dass seine Tragstruktur im aufgeweiteten Zustand eine ausreichende Tragkraft aufweist, um das Gefäß offen zu halten. Um unnötige Gefäßbeschädigungen zu vermeiden, ist außerdem gewünscht, dass der Stent nach dem Aufweiten und nach Entfernen des Ballons nur wenig elastisch zurückfedert (Recoil), so dass der Stent beim Aufweiten nur wenig über den gewünschten Enddurchmesser hinaus geweitet werden muss. Weitere Kriterien, die in Bezug auf einen Stent wünschenswert sind, umfassen beispielsweise eine gleichmäßige Flächenabdeckung und eine Struktur, die eine gewisse Flexibilität in Bezug auf die Längsachse des Stents erlaubt.

In einigen Fällen ist eine dauerhafte Stützfunktion durch den Stent nicht erforderlich; das Körpergewebe kann sich unter Anwesenheit des Stents derart erholen, dass eine Stützwirkung durch den Stents nicht mehr notwendig erscheint. Dies hat zu dem Gedanken geführt, Stents aus bioresorbierbarem Material zu fertigen. In Bezug auf einen biodegradierbaren Metallstent ist es über die vorgenannten Anforderungen hinaus wünschenswert, wenn von den Abbauprodukten des Metallstents möglichst keine oder nur sehr geringe negative physiologische Wirkungen ausgehen.

Neben den gewünschten mechanischen Eigenschaften eines Stents soll dieser möglichst in einer Weise mit dem Körpergewebe am Implantationsort interagieren, dass es nicht zu erneuten Gefäßverengungen, insbesondere durch den Stent selbst hervorgerufenen Gefäßverengungen kommt. Eine Restenose (Wiederverengung des Gefäßes) soll möglichst vermieden werden. Derzeit werden bei etwa 70% aller perkutanen Interventionen Stents eingesetzt, in 25% aller Fälle kommt es jedoch zu einer in-Stent Restenose aufgrund eines überschießenden neointimalen Wachstums, das durch eine starke Proliferation der arteriellen glatten Muskelzellen und eine chronische Entzündungsreaktion hervorgerufen wird. Zur Senkung der Restenoseraten werden die verschiedensten Ansätze verfolgt, wie z. B. eine intrakoronare radioaktive Bestrahlung (Brachytherapie).

Ein weiterer Ansatz zur Verbesserung des Restenoseproblems sieht vor, den Stent mit einer geeigneten pharmazeutisch aktiven Substanz (Wirkstoff) zu beschichten, entweder durch direkte Anbindung des Wirkstoffs an die Stentoberfläche oder Einbettung desselben in eine auf der Stent-oberfläche aufgebrachte Trägermatrix. Beispiele umfassen die Wirkstoffe Sirolimus und Paclitaxel. Die direkte Anbindung von Wirkstoffen an der Stentoberfläche hat sich als wenig praktikabel erwiesen; ganz überwiegend wird der Wirkstoff in einer Trägermatrix bereitgestellt.

Als Trägermatrix können beispielsweise synthetische Polymere (z. B. Polyurethane, Polymethacrylate, Polyvinylalkohole), degradierbare Polymere (z. B. Polyhydroxybuttersäure, Polylactide) oder Polymere rein biologischen Ursprungs (z. B. Hyaluronsäure, Phosphorylcholin) verwendet werden. Ein Teil der Polymere ruft jedoch starke Entzündungsreaktionen hervor und induziert damit den Prozess der Restenose. Es wurde von Fällen subakuter Thrombosen und allergische Reaktionen berichtet, die vermutlich durch die für die Trägermatrix verwendeten Polymere hervorgerufen werden. Besonders Patienten mit multipler oder schwieriger Symptomatik (wie Diabetes, komplexe Läsionen, kleine Gefäße, lange Läsionen) zeigen eine erhöhte Thromboserate. Weiter sind aus US 2006/0030936 und WO 01/15752 Cholesterolbeschichtungen und aus US 2004/0224003 Beschichtungen auf Ölbasis für medizinische Implantate bekannt, die eine oder mehrere pharmazeutisch aktive Substanzen enthalten.

Inwiefern die Komponenten einer Trägermatrix beim Einsatz in vivo tatsächlich den gewünschten Kriterien der Verträglichkeit genügen, lässt sich allein anhand von Literaturdaten nicht hinreichend genau prognostizieren. Gerade bei biodegradierbaren Implantaten ist zudem eine Interaktion mit den Addukten bzw. den reaktiven Komponenten des Grundkörpers zu beachten. Allein das Auffinden eines für die Beschichtung geeigneten Materials erfordert daher ein hohes Maß an Verständnis der zugrunde liegenden biologischen Mechanismen, die Kenntnis der gewünschten Materialeigenschaften hinsichtlich der Verarbeitung und späteren Verwendung als auch Kenntnis über die Verfügbarkeit und die möglichen Kosten, die mit dem Einsatz des Materials verbunden sind. Das Finden eines solchen Materials ist demnach sehr aufwändig und kann nicht in standardisierter Weise erfolgen, gerade auch deshalb, weil viele Materialeigenschaften, die für den Verwendungszweck eine Rolle spielen können, noch nicht beschrieben oder voraussehbar sind und erst in aufwändigen Versuchen belegt werden müssen.

Es besteht demnach ein Bedarf nach zumindest weiteren alternativen Beschichtungsmaterialien für die Freisetzung von hydrophoben Wirkstoffen für biokorrodierbare Implantate, insbesondere Stents.

### Zusammenfassung der Erfindung

Die Erfindung liegt in der Bereitstellung eines Implantats, welches einen Grundkörper aus einer biokorridierbaren Legierung und eine zumindest bereichsweise Beschichtung der Außenoberfläche aufweist, welche ein oder mehrere Komponenten ausgewählt aus der Gruppe Cholesterol und Cholesterolester und ein oder mehrere pharmazeutisch aktive Substanzen enthält.

Cholesterol (Cholest-5-en-3β-ol; Cholesterin) ist eine farblose Substanz mit einem Schmelzpunkt von 148,5°C. Cholesterol ist in Wasser praktisch nicht, in Alkohol in der Kälte wenig, in der Wärme besser, in Ether, Benzol, Petrolether löslich. Als Hauptvertreter der Zoosterole ist Cholesterol in allen Organen verbreitet: Im Großhirn (etwa 10% der Trockensubstanz), in Nervenzellen, Nebennieren und Haut. Das Blut enthält 0,15 - 0,25%, das Herz 2% Cholesterol. Insgesamt enthält der menschliche Körper durchschnittlich 0,32% Cholesterol, teils frei, teils mit Fettsäuren verestert. Täglich werden im Körper des Erwachsenen ca. 1 -2 g Cholesterol synthetisiert. Hauptbildungsort ist die Leber, aber auch in der Nebennierenrinde, in Haut, Darm, Testes (Hoden) und Aorta wird Cholesterol gebildet. Der Transport des Cholesterol und seiner Ester im Blut erfolgt in Form von Lipoproteinen. Das zu den Lipiden gerechnete Cholesterol ist neben Phospho- und Glycolipiden wichtiger Bestandteil von Biomembranen, insbesondere der Plasmamembranen von Eukaryonten, deren Fluidität es reguliert. Cholesterol spielt im Organismus auch eine Rolle als Hautschutzsubstanz, Quellungsregulator, Nervenisolator und dergleichen. Durch falsche Ernährung, aber auch durch bestimmte Enzym- oder Rezeptordefekte können pathologisch erhöhte Cholesterol-Spiegel im Serum entstehen (Hypercholesterinämie). Diese erachtet man als mitverantwortlich für die Entstehung von Arteriosklerose, bei der sich Cholesterol-reiche Ablagerungen an Arterienwänden bilden. Cholesterol findet Verwendung als Emulgator für kosmetische und pharmazeutische Präparate, Textilwaren, Lederpflegemittel und dergleichen, Bestandteil von Haarwuchsmitteln, Ausgangsmaterial für die Vitamin-D-Synthese and anderer Steroide sowie für die als flüssige Kristalle wichtigen Cholesterolester.

Als einwertiger, sekundärer Alkohol vermag Cholesterol Ester (Cholesterolester; Cholesterinester; Cholesterylester) zu bilden, insbesondere mit aliphatischen oder aromatischen Carbonsäuren wie Öl-, Palmitin-, Stearin-, Benzoe-, Linol- oder Zimtsäure. Im Lipidstoffwechsel stellen Cholesterolester eine Speicher- und Transportform des Cholesterols dar. Ihre Bildung erfolgt extrazellulär unter Katalyse durch Lecithin: Cholesterol-Acyltransferase (EC 2.3.1.43), ihre Speicherung in Lipoproteinen.

Es hat sich nun überraschenderweise gezeigt, dass Cholesterol bzw. seine Ester vorteilhafte Komponenten einer Implantatsbeschichtung, insbesondere für Stents, sein können. Aus der Sicht der Anmelderin ist die Eignung von Cholesterol(-estern) umso überraschender, als dass Cholesterol bekanntermaßen eine tragende Rolle bei der Bildung von Gefäßerkrankungen spielt und gerade im Bereich dilatierter Läsionen häufig Cholesteroleinlagerungen in den Gefäßwänden zu finden sind. Die besondere Eignung des Cholesterols bzw. seiner Ester mag darin begründet sein, dass es als körperidentisches Produkt oder Homologon keine Abstoßungsreaktionen bei der Freisetzung bzw. beim Kontakt mit Körpergewebe verursacht. Die sehr geringen Mengen an Cholesterol(estern) haben keinen oder allenfalls einen sehr geringfügigen Effekt auf die Läsion, so dass die Vorteile der Verwendung der Substanzen weit überwiegen. Nur vordergründig erscheint demnach die bekannte Bedeutung der Hypercholesterinämie als etablierter Risikofaktor der Atherogenese limitierend. Im Kontext koronarer Interventionen konnte bislang keine Assoziation von Serumlipiden und der Restenosierung nach alleiniger PTCA oder auch Stentimplantation gezeigt werden.

Ein besonderer Vorteil der Verwendung von Cholesterol und/oder von Cholesterolestern liegt darin, dass sie aufgrund ihres hydrophoben Charakters als Trägermatrix für hydrophobe Wirkstoffe, wie z. B. Paclitaxel, Pimecrolimus oder Sirolimus, agieren können. Die Beschichtung enthält somit vorzugsweise zusätzlich eine oder mehrere pharmazeutisch aktive Substanzen, insbesondere hydrophobe pharmazeutisch aktive Substanzen. Daneben können natürlich auch nicht hydrophobe Wirkstoffe Einsatz finden. Die Wirkstoffe umfassen insbesondere Substanzen zur Behandlung der Instent-Restenose, zur Behandlung von Nebeneffekten bei Stentimplantation und Substanzen, die den Heilungsverlauf nach Implantation unterstützen.

Eine Beschichtung im Sinne der Erfindung ist eine zumindest abschnittsweise Auftragung der Komponenten auf den Grundkörper des Implantats. Ist das Implantat ein Stent, so umfasst der Grundkörper des Stents die baulichen Strukturen, die die mechanischen Eigenschaften des Stents für die bereits oben genannten Zwecke gewähren. Vorzugsweise wird die gesamte Oberfläche des Grundkörpers des Stents von der Beschichtung bedeckt. Nach einer weiteren Variante kann die Beschichtung als eine Vertiefung oder Bohrung im Implantatskörper sein, die mit dem Material aufgefüllt ist, insbesondere im Zusammenspiel mit pharmazeutisch aktiven Substanzen.

Implantate umfassen - neben Stents - vorzugsweise orthopädische Implantate wie Schrauben und Platten, Hüftgelenke, Herzklappen, Knochenimplantate, Bypässe, Elektroden sowie Defibrillator- und Schrittmachergehäuse. Denkbar ist auch, das Implantat als so genanntes Kurzzeitimplantat einzusetzen, z.B. in Form eines beschichteten Katheters, beschichteten Führungsdrahtes oder beschichteter Elektroden. Die Implantate werden ganz oder abschnittsweise mit der erfindungsgemäßen Beschichtung versehen.

Die Beschichtung enthält Cholesterol bzw. Cholesterolester vorzugsweise als Hauptbestandteile. Ein Hauptbestandteil im Sinne der Erfindung ist eine Komponente der Beschichtung, deren Gewichtsanteil am Gesamtgewicht der Beschichtung am größten ist. Insbesondere liegt der Gewichtsanteil der Hauptkomponente bei mindestens 50 Gew.%, besonders bevorzugt mindestens 70 Gew.%. Für den Fall, dass die Beschichtung Cholesterol und einen oder mehrere Cholesterolester enthält, so liegt die Summe der Gewichtsanteile dieser Komponenten vorzugsweise bei mindestens 50 Gew.%, insbesondere bei mindestens 70 Gew.%.

Vorzugsweise enthält die Beschichtung zusätzlich Weichmacher wie Linolsäure oder Tocopherol, insbesondere in Kombination mit Cholesterol (nicht mit Cholesterolestern). Die Beimengung von Linolsäure erhöht die Geschmeidigkeit des Beschichtungsmaterials und erleichtert die Verarbeitung und Aufbringung desselben auf dem Implantat, insbesondere Stent. Vorzugsweise liegt dabei ein Gewichtsverhältnis von Linolsäure zu Cholesterol im Bereich von 1 : 3 bis 1 : 20.

Ferner ist bevorzugt, dass der Cholesterolester Cholesterol-linolat ist, d.h. ein Ester aus Cholesterol und Linolsäure. Dieser Ester ist aufgrund seines Schmelzpunktes, der laut Literaturangaben im Bereich von 38 bis 41°C liegt, besonders geeignet für den Einsatz im menschlichen Körper, da das allmähliche Erweichen der Substanz bei 37°C Körpertemperatur z. B. ein Absplittern der Beschichtung während der Stentexpansion verhindert und die Beschichtung auch nach der Verformung gleichmäßig die Stentoberfläche bedeckt. Letztere Eigenschaft ist insbesondere im Zusammenhang mit biodegradierbaren Grundkörpern von besonderer Bedeutung, denn Fehlstellen in der Beschichtung stellen Angriffspunkte der Grundkörperkorrosion dar, mit der Folge, dass die Degradation des Implantats unkontrolliert ablaufen kann. Enthält die Beschichtung eine Kombination aus Cholesterol-linolat und Cholesterol, so liegt vorzugsweise ein Gewichtsverhältnis des Esters zum Alkohol im Bereich von 1 : 3 bis 1 : 20.

Daneben kann die Beschichtung folgende Zusatzstoffe enthalten:
- Lipophile Vitamine (Vitamin A, D, E, K)
- weitere Fettsäuren außer Linolsäure (Öl-, Palmitin-, Stearin-, Benzoe-, Zimt-, Linolen-, Arachidon-, Myristin-, Arachin-, Behen-, Palmitolein-, Elaidin-, Vaccen-, Icosen-, Cetolein-, Eruca-, Nervonsäure)
- Antioxidantien (Alpha-Tocopherol E 307, Ascorbinsäure E 300, Ascorbylpalmitat E 304, Butylhydroxytoluol (BHT) E 321, Butylhydroxyanisol (BHA), Calcium-Dinatrium-EDTA E 385, Calcium-L-Ascorbat E 302, Calciumhydrogensulfit E 227, Calciumsulfit E 226, Citronensäure E 330, Delta-Tocopherol E 309, Diphosphate E 450, Dodecylgallat, Laurylgallat E 312, Gamma-Tocopherol E 308, Isoascorbinsäure E 315, Kaliumbisulfit E 228, Kaliumcitrat E 332, Kaliumsulfit E 224, Lecithin E 322, Milchsäure E 270, Natrium-L-Ascorbat E 301, Natrium-L-Ascorbat E 301, Natriumbisulfit E 222, Natriumcitrat E 331, Natriumdisulfit E 223, Natriumisoascorbat E 316, Natriumsulfit E 221, Octylgallat E 311, Polyphosphate E 452, Propylgallat E 310, Schwefeldioxid E 220, Tocopherol E 306, Triphosphate E 451, Zinn-II-Chlorid E 512)
- Emulgatoren (Ammoniumphoshatide E 442, Ascorbylpalmitat E 304, Calciumphosphat E 341, Calciumstearoyl-2-lactylat E 482, Citronensäureester von Mono- und Diglyceriden von Speisefettsäuren E 472c,

Diphosphate E 450, Kaliumphosphat E 340, Lecithin E 322, Natriumphosphat E 339, Natriumstearoyl-2-lactylat E 481, Phosphorsäure E 338, Polyglycerin-Polyricinoleat E 476, Polyoxyethylen (40) stearat E 431, Polyphosphate E 452, Polysorbat 20 E 432, Polysorbat 40 E 434, Polysorbat 60 E 435, Polysorbat 65 E 436, Polysorbat 80 E 433, Propylenglycolalginat E 405, Sorbitanmonolaurat E 493, Sorbitanmonooleat E 494, Sorbitanmonopalmitat E 495, Sorbitanmonostearat E 491, Sorbitantristearat E 492, Stearyltartrat E 483, Triphosphate E 451, Zuckerglyceride E 474)
- Phospholipide
- Fluoreszenzmarker
- Röntgenmarker
- Kontrastmittel für magnetische Resonanzaufnahmen
- Farbstoffe

Bevorzugt wird als Zusatzstoff ein Tocopherol oder ein Tocopherol-Derivat beigesetzt. Vorzugsweise liegt dabei ein Gewichtsverhältnis von Cholesterol(ester) zum Tocopherol(derivat) im Bereich von 3 : 1 bis 1 : 1.

Nach einer bevorzugten Ausführungsform besteht das Implantat / der Stent ganz oder in Teilen aus einer biokorrodierbaren metallischen Legierung, insbesondere Magnesiumlegierung. Das Implantat weist also einen Grundkörper aus der biokorrodierbaren metallischen Legierung auf, dessen Außenoberfläche zumindest bereichsweise die Beschichtung trägt. Biokorrodierbar bedeutet, dass der Werkstoff nach Implantation allmählich, z. B. durch hydrolytische oder enzymatische Prozesse, abgebaut wird. Derartige Legierungen sind beispielsweise aus EP 1 419 793 A1 bekannt, auf deren Offenbarungsgehalt bezüglich der verwendeten Magnesiumlegierungen verwiesen wird. Der Einsatz von Cholesterol und/oder Cholesterolestern als Beschichtungsmaterial für Implantate aus einer biokorrodierbaren metallischen Legierung, insbesondere Magnesiumlegierung, ist deshalb besonders bevorzugt, weil die Beschichtungsmaterialien bekanntlich hydrophob sind und damit eine Beschichtung des Implantats die Abbauprozesse hemmt/verzögert. Mit anderen Worten, durch eine derartige hydrophobe Beschichtung kann das Degradationsverhalten des Implantats beeinflusst werden. Beispielsweise kann durch Variation der Beschichtungsdicke in unterschiedlichen Bereichen des Implantats Einfluss auf das lokale Degradationsverhalten des Implantats genommen werden. Neben dieser bevorzugten Ausführungsform sind natürlich auch Beschichtungen auf permanenten metallischen oder polymeren Implantaten sowie Beschichtungen auf degradierbaren polymeren Implantaten denkbar.

Die Erfindung wird nachfolgend anhand eines Ausführungsbeispiels näher erläutert.

### Ausführungsbeispiel 1 - Beschichtung eines biodegradierbaren Stents

Ein Grundkörper des zu beschichtenden Stents bestand aus der biodegradierbaren Magnesiumlegierung WE43.

Bei Raumtemperatur wurde eine Lösung von 0,2 g Cholesterol und 0,2 g alpha-Tocopherol in 3 ml Cyclohexan angesetzt. Der Stent wurde in die angesetzte Lösung getaucht, wieder entnommen und bei Raumtemperatur getrocknet.

Beschichtete Stents wurden ins Schwein implantiert. Eine Explantation erfolgte nach 35 Tagen. Erste histologische Begutachtungen zeigten, dass das Ausmaß der Restenose gegenüber unbeschichteten Stents deutlich verringert ist.

### Ausführungsbeispiel 2 - Stentbeschichtung mit Pimecrolimus

Ein Grundkörper des zu beschichtenden Stents bestand aus der biodegradierbaren Magnesiumlegierung WE43.

Bei Raumtemperatur wurde eine Lösung von 0,3 g Cholesterol, 0,1 g Linolsäure und 0,1 g Pimecrolimus in 12 ml Chloroform angesetzt. Der Stent wurde in die angesetzte Lösung getaucht, wieder entnommen und bei Raumtemperatur getrocknet.

## Patentansprüche

1. Implantat aufweisend einen Grundkörper aus einer biokorrodierbaren metallischen Legierung und eine zumindest bereichsweise Beschichtung der Außenoberfläche, welche ein oder mehrere Komponenten ausgewählt aus der Gruppe Cholesterol und Cholesterolester und ein oder mehrere pharmazeutisch aktive Substanzen enthält.

2. Implantat nach Anspruch 1, bei dem die biokorrodierbare metallische Legierung eine Magnesiumlegierung ist.

3. Implantat nach Anspruch 1, bei dem die Beschichtung zusätzlich Linolsäure enthält.

4. Implantat nach Anspruch 3, bei dem ein Gewichtsverhältnis von Linolsäure zu Cholesterol im Bereich von 1 : 3 bis 1 : 20 liegt.

5. Implantat nach Anspruch 1, bei dem der Cholesterolester Cholesterol-linolat ist.

6. Implantat nach einem der Ansprüche 1 bis 5, bei dem das Implantat ein Stent ist.

## Claims

1. An implant comprising a base body composed of a biocorrodible metallic alloy and an at least partial coating of the outer surface, which contains one or more components selected from the group cholesterol and cholesterol ester, and one or more pharmaceutically active substances.

2. The implant according to claim 1, wherein the biocorrodible metallic alloy is a magnesium alloy.

3. The implant according to claim 1, wherein the coating also contains linoleic acid.

4. The implant according to claim 3, wherein a weight ratio of linoleic acid to cholesterol is in the range of 1:3 to 1:20.

5. The implant according to claim 1, wherein the cholesterol ester is cholesterol linoleate.

6. The implant according to one of the claims 1 through 5, wherein the implant is a stent.

## Revendications

1. Implant comportant un corps de base constitué d'un alliage métallique biocorrodable, avec un revêtement au moins partiel sur la surface extérieure, contenant un ou plusieurs composants sélectionnés à partir du groupe de cholestérol et ester Cholestérol, ainsi qu'une ou plusieurs substances pharmaceutiques actives.

2. Implant selon la revendication 1, dans lequel l'alliage métallique biocorrodable est un alliage de magnésium.

3. Implant selon la revendication 1, dans lequel le revêtement contient en outre de l'acide linoléique.

4. Implant selon la revendication 3, dans lequel le rapport en poids de l'acide linoléique par rapport au cholestérol est compris entre 1:3 et 1:20.

5. Implant selon la revendication 1, dans lequel le ester de cholestérol est du linolate de cholestérol.

6. Implant selon l'une des revendications 1 à 5, dans lequel l'implant est un stent.
